# EUROPEAN PATENT APPLICATION

(11) **EP 3 011 980 A1**
(43) Date of publication of application: **27.04.2016**
(21) Application number: 14306697.5
(22) Date of filing: 24.10.2014
(51) Int. Cl.: A61L 27/06, A61L 27/56, A61F 2/20

(54) **Method for treating titanium prosthesis**

(71) Applicant: Protip Medical, 67000 Strasbourg (FR)
(72) Inventor: Vrana, Nihal Engin, 67100 STRASBOURG (FR); Perrin, Nicolas, 67300 SCHILTIGHEIM (FR); Berenger, Maurice, 26740 SAVASSE (FR)
(74) Representative: Flesselles, Bruno F.G.

(57) **Abstract**

The invention relates to tubular prostheses or implants having increased protein adsorption on the external surface and not on the internal surface, and to the process of obtaining such prosthesis by specific surface treatments on localized parts of said prosthesis.

## Description

The invention relates to the field of surface treatment of prostheses or implants.

Titanium is a commonly used biomaterial with a wide range of biomedical applications such as dental and hip implants (Li *et al*.). In these applications, it is important to ensure a good contact with the titanium surface and the host tissue. Most of the studies on titanium focused on its interaction with bone tissue cells or stem cells with an aim to convert them to osteoblasts.

Recently there have been attempts to use titanium implants in other medical indications, particularly for tracheal replacement (Vrana *et al.,* 2012; Janssen *et al.;* Dupret-Bories *et al.;* Gaafar *et al.).* These efforts necessitate porous titanium structures that need to be colonized by the connective tissue of the host. In turn, use of such structures requires a better understanding of the interaction of titanium surfaces with soft tissues (i.e. the tissues that connect, support, or surround other structures and organs of the body, not being bones), in particular to determine the ability of cells of such tissues to colonize said implants.

The behavior of different cell types in contact with titanium can be different. One such example is the interaction of gingival epithelial cells with dental titanium implants where epithelial cells prefer polished titanium to sandblasted titanium (Lauer *et al*.). However, this is not necessarily applicable to all cases and the cellular interaction with surfaces of porous titanium structures can be considerably different.

Cell/implant interactions are further different for otorhinolaryngology and laryngeal replacement, as unlike in the case of dental implants where most of the patients are healthy otherwise, tracheal implants are designed particularly for patients with recent cancer and surgical history. Hence, it is even more important to optimize surface properties for quick and efficient implant tissue integration.

In the case of larynx or tracheal implants, the implant needs to be tubular, or at least present an internal open lumen, to enable normal breathing. The inner part of the implant will be in contact with air and will be unsterile whereas the exterior part of the implant will be contacting the host connective tissue and thus needs to interact with it. This would thus preferably require different treatments of the exterior part and the interior part of the implant as the surface treatments that can help the integration of the implant may create problems if they are also applied to the lumen of the implant. These problems would include faster attachment and biofilm formation by bacteria, restenosis due to uncontrolled cellular movement into the lumen and subsequent clogging of the lumen etc. Thus for such applications differential treatment of the different regions of the implant is preferred.

Removal of larynx is a necessity for late stage laryngeal cancer patients and it results in considerable problems for the patient due to the presence of a tracheostomy (the trachea is attached to the skin of the neck to re-establish breathing.). The most important consequences are due to the shunt of the aero digestive upper way: breathing with the tracheostomy (and not with the nose or the mouth), and difficulties in speaking (the patient has to learn oesophageal voice, or a small valve between the trachea and the oesophagus can be placed during the surgery). An artificial larynx system that can replace the functions of the larynx after total larynx resection would significantly improve the life quality of these patients. A system composed of a removable valve system and a permanent titanium implant part (which is developed by the Applicant and has been described in various patent applications such as WO 2013/079362 and WO 2013/034858) can restore several functions of larynx and provides the patient with a means to breathe without a tracheostomy. Titanium was selected because the mechanical properties of titanium prevent the possible collapse of the airway, which is a common risk in tracheal implants.

In order to ensure the proper integration between the implant and the trachea, a macroporous, microbead based system was utilized. However, due to the physical properties of the titanium, the connection between the titanium and the surrounding tissue might not be rapidly established, for the permanent titanium implant. This would further lengthen the inflammatory process and the duration between the two steps of implantation. Thus, it is important to mediate the interaction of the implant with the surrounding soft tissues via surface modifications without compromising the bulk properties of the implant. Furthermore, obtaining a tight junction of the surrounding tissue and of the implant would also help prevent bacteria, saliva and food bolus to enter the trachea at the junction between it and the tracheal implant.

Surface properties are an important aspect of non-degradable, implantable biomaterials (Variola *et al*.) and are widely used to improve metallic implant's bioactivity. The acid etching is a widely used technique for improving the interaction between titanium implants with cells. Primarily developed for dental implants, acid etching has been shown to significantly improve the integration of implants with surrounding tissue. However, the literature on this effect related to soft tissue are relatively rare, and its use in the Otorhinolaryngology field implants are not available.

For example, it has been shown that acid etching of orthopaedic implants resulted in better osseointegration as quantified by the increased torque force to remove the implants *in vivo* (Cho *et al*.). Many methods, such as hydroxyapatite coating, polymer adsorption, etching, covalent bonding, anodization have been used to achieve beneficial effects (Zhao *et al.,* Mustafa *et al*.).

In dental implants field, the general understanding is that a rough titanium surface is better for bone attachment whereas a smooth surface helps the gingival fibroblast adhesion (Kunzler *et al*.). However, these conclusions are based on a structure where the interaction of the cells with the implant surface is planar.

This approach cannot fully capture the possible *in vivo* outcomes for implants with different parts facing different tissues, particularly for porous implants. Moreover, it is difficult to pinpoint the exact reasons behind the improvements in cellular behavior.

How the surface properties of titanium in a 3D configuration (in particular porous titanium) affect the cellular interaction with the implant surface is still an open question. These effects are difficult to quantify in metal foams as the pore shapes are irregular and the distribution of the pore sizes are widespread.

Microbead based macroporous titanium implants, provides a 3D system where it is possible to study the effects of surface modification on the building blocks of the system (Vrana *et al.,* 2013a). It has been shown that extensive changes in the *in vivo* integration process can be obtained just by changing the size of microbeads (Vrana *et al.,* 2013b). It has also been previously shown that HCl etching of porous titanium implants improves their osseoinductivity (Takemoto *et al*.). Furthermore, effects of anodisation on osseointegration and osteogenic differentiation of mesenchymal stem cells have also been demonstrated (Lavenus *et al.,* 2011 and 2012).

The present application reports the fact that modifying the surface of titanium implants, in particular by an acid etching treatment or an anodization treatment would improve the *in vivo* integration of this implant, in particular in the trachea. These treatments induce, *inter alia,* modifications of the protein adsorption, hydrophilicity and actual surface structure of the implants.

The methods and implants described herein can be used for any type of prosthesis having an internal lumen where the external face of said prosthesis is to be integrated in a tissue and where a fluid is intended to flow within the internal face (lumen) of this prosthesis, which should thus be such that colonization by cells is to be prevented.

In the context of the present application, the term "tubular" shall represent a geometrical shape that presents a well-defined lumen. A tubular implant (or prosthesis) will thus present an internal face (lumen) and an external face. It can be of any shape, as long as it presents said external, internal faces and lumen. It can be cylindrical or of any other shape. It is nevertheless preferred when it is in the form of a cylinder (open at both ends) or of a tube.

The invention thus related to a prosthesis comprising a tubular part made of titanium, wherein said tubular prosthesis presents protein adsorption on the external face of said tubular part that is higher that the protein adsorption on the internal face of said tubular part.

In a preferred embodiment, said tubular part is made in whole of porous titanium.

In another embodiment, said tubular part is made in whole of solid titanium.

In another embodiment, said tubular part is made porous and of solid titanium.

It is to be noted that the prosthesis may contain other tubular parts, made of titanium (whether porous or nor), which do not present the above characteristic.

In the context of the present application, a prosthesis (which may also be called "implant") relates to medical device intended to be implanted in the body of a patient, and which is used in a process of replacing a missing body part and/or restore a lost or impaired functionality. In particular, the prosthesis used in the above method may not necessarily be able, by itself, to replace said missing body part or replace said functionality, but would then be used in a process intended to achieve said purpose.

In a first embodiment, said prosthesis comprises a part in porous titanium. In this embodiment, said prosthesis may comprise a part made of porous titanium (especially microporous titanium) and a part made of plain titanium. The prosthesis may also contain parts made of other materials such as silicone.

In another embodiment, said prosthesis comprises a part made of porous titanium and a part made of another material than titanium.

In another embodiment, the prosthesis comprises a part made of plain titanium and a part made of another material than titanium.

It is preferred when said porous titanium is obtained through consolidation of microspheres of titanium, of titanium-based alloys, I.e. by consolidating the microspheres with each other as well as with said at least one solid core by electrical discharge sintering.

Microporous titanium is made up of a three-dimensional juxtaposition of plain titanium spheres (see standard NF ISO 5832-2 dated March 1997) and the prosthesis may be obtained, in particular according to the methods described in EP 856299, WO 2007/048935 or WO 2007/034077. According to the method of EP 856299, a metallic prosthesis using microporous titanium has an open porosity characterized by interspheroid spaces with a dimension approximately equal to one third of the diameter of the powders, i.e. comprised between 50 and 150 µm for powders from 150 to 500 µm.

The method for preparation of the metallic prosthesis according to one of the embodiments described in EP 856299 implies the implementation of the several successive stages as follows:
- obtaining by pulverization with a rotating electrode (or any other method giving spheres with low or no bumps) of a powder made up of metallic microspheres of titanium or of titanium based alloy metallic microspheres or of any other biocompatible alloy,
- possible gauging of the microspheres by sifting,
- forming the prosthesis by heat treatment aiming to interlock said microspheres at the level of their contact areas in a mould of appropriate shaped.

The method of WO 2007/048935 involves the following steps:
- selecting a mould, nonconductive, of suitable shape corresponding to the desired implant,
- arranging in said mould at least one solid metal core,
- filling out the volume of the mould remaining available with a powder of microspheres, and
- consolidating the microspheres with each other as well as with said at least one solid core by electrical discharge sintering,
- said stage of electrical discharge sintering being realized by discharge of an electrical current, of a predetermined voltage in order to obtain an energy surface density J generally lower or equal to 10 J/mm² between on one hand, at least a first electrode at the periphery of the mould and on the other hand, at least a second electrode of opposite polarity and positioned so as the lines of current formed between these two electrodes are not parallel to the microspheres/metallic core interface of higher surface.

In the context of the claimed method, it is preferred when the size of the titanium microbeads used to make the microporous titanium is comprised between higher than 100 µm, preferably higher than 150 µm or than 200 µm, but lower than 500 µm, preferably lower than 400 µm, preferably around 300 µm.

This would lead the a microporous titanium with pores having an average size higher than 50 µm, preferably higher than 75 µm but lower than 200 µm preferably lower than 166 µm or than 150 µm, preferably around 100 µm.

"Around" is to be understood as +/- 10%.

In the context of the present invention, protein adsorption is measured by BCA assay, as described below:

In order to measure protein adsorption on the external face of the implant, one would apply the protein solution (preferably human or bovine serum) on to the exterior surface by dipping for 1 h, at 37°C where the interior surface is inaccessible due to the presence of a mask for this inner surface.

After 1 hour of incubation the sample is washed with PBS once and then dipped into a detergent solution to remove the adsorbed proteins. This solution is composed of 3M Urea/5% sodium dodecyl sulfate (SDS) and the desorption is done by overnight treatment.

BCA assay (bicinchoninic acid) is a detergent compatible total protein determination method. In order to estimate the total amount of protein adsorbed in terms of protein amount, a calibration curve with known protein concentrations (from 0 µg/ml to 2000 µg/ml) (preferably with bovine serum albumin (BSA)) is first established.

Then, the amount of protein inside the desorption solution was determined by incubating 10 µl of the desorption solution (which contains the proteins adsorped on the implant surface) in 200 µl of BCA reagent solution at 37 °C for 30 minutes and measuring the absorbance at 562 nm with a microplate reader.

In order to measure protein adsorption on the internal face of the implant, one will fill the inside of the implant with the protein solution, preferably human or bovine serum albumin and close the top and bottom parts with the caps of the holder. After 1 hour of incubation, the solution is removed the inside is washed with PBS and the proteins are desorbed as described above. The measure of the amount of protein in the desorption solution (which is the amount of protein absorped on the inner surface) is obtained by the BCA assay as described above.

In particular, it is preferred when the protein adsorption on the external face is higher of at least 10 %, more preferably of at least 15 %, most preferably of at least 20 % than the protein adsorption on the internal face of the tubular part of the implant.

The prosthesis may also present an enhanced hydrophilicity on its external face. In particular, it is preferred when said external face of the tubular part exhibits a contact angle lower than 65 °, more preferably lower than 60° as measured for a 10 microliter water droplet.

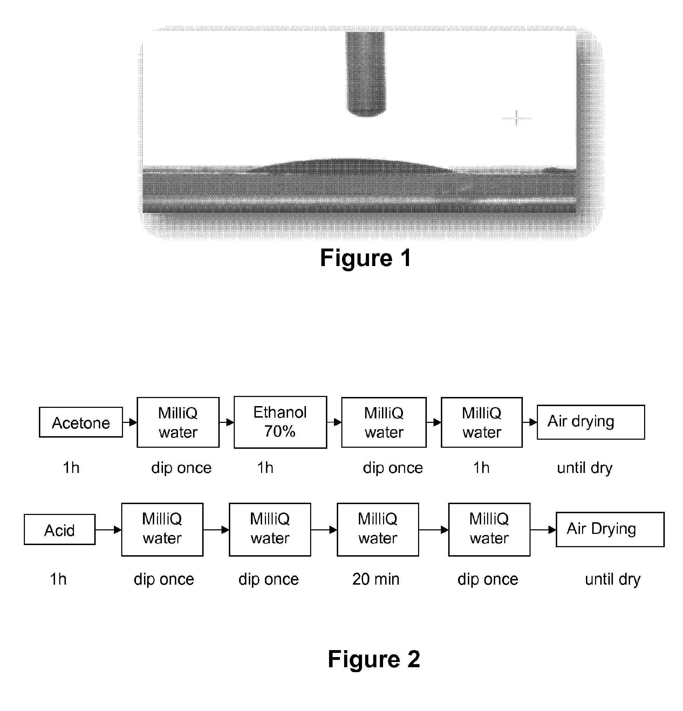

Surface hydrophilicity of the implant is measured by determination of the water contact angle (the Sessile drop method) using a goniometer on implant which have been blotted dry (Figure 1). Implants are positioned on a stage and fixed in place under the syringe. The syringe tip position is arranged by the help of a video camera and a 10 microliter water drop was applied onto the surface. After the drop is in place, pictures are taken by an on-board camera of the goniometer, showing the contact point of the water drop with the surface. The angle measurements is made automatically after the contact of the drop with the implant.

In comparison, the inner face of the implant would generally exhibit a contact angle higher than 65°.

The nanostructure of the surface of the external face of the implant is modified with regards to the structure of the surface of the inner face.

In particular, said prosthesis will generally exhibit a quantifiable decrease (at least 50%) in machining marks on the surface of the exterior face of the tubular part and no changes on the surface of the interior part, as measured by microscopic image analysis methods.

The machining marks are easily identifiable with light microscopy and the implants are regularly analyzed by imaging the exterior marks before and after surface treatment (etching, anodization) in different regions.

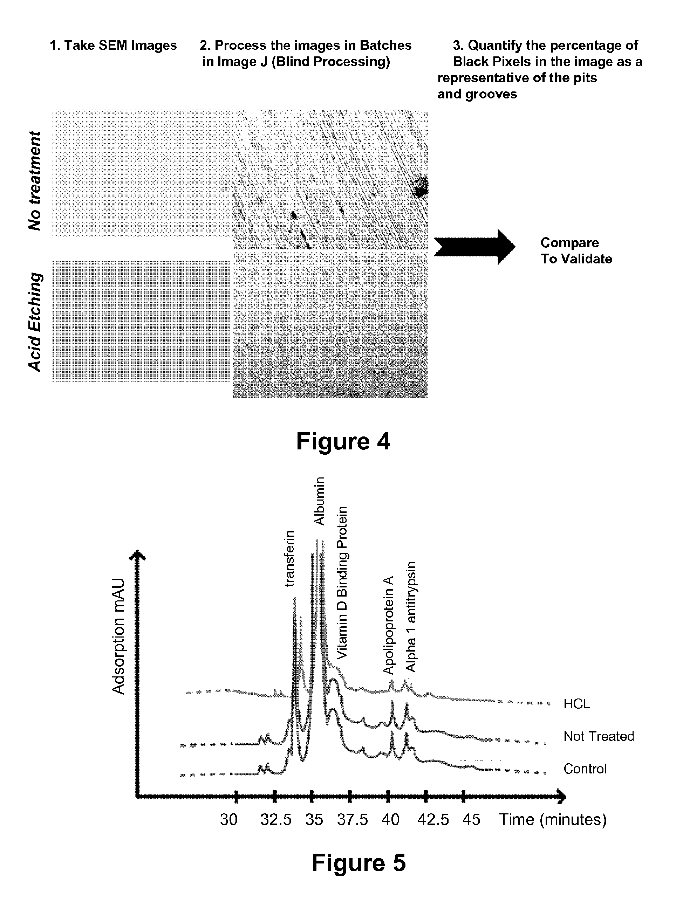

A proper surface treatment cycle can be judged by a substantial decrease in the marks (defined as the removal of specific lines from the surface and replacement with a smooth, slightly wavy surface features) either by operator judgment with respect to pre-set reference pictures or via image analysis (see Figure 4).

As indicated above, and although the prosthesis may be used for any purpose when it is necessary to have a fluid flow within the prosthesis, it is particularly preferred when the prosthesis is a tracheal or laryngeal prosthesis. A tracheal or laryngeal prosthesis is an implant that is intended to be implanted in patients having undergone ablation of the larynx, preferably in the trachea of said patients. The length of this implant is thus preferably at least 40 mm, preferably at least 45 mm, but at most 80 mm, preferably at most 70 mm, generally around 50 or 65 mm. The internal diameter of the implant is generally between 12 and 20 mm and the external diameter between 18 and 29 mm. The implant dimensions can be adapted if the specific anatomy of a patient necessitates such a change.

In a preferred embodiment, the prosthesis consists in a medical device for supporting an implant or prosthesis, wherein said device comprises two zones, one of which constituting an upper ring made of a rigid or semi-rigid, solid titanium, and the other of which constituting a lower ring made of a rigid or semi-rigid, porous titanium.

In such embodiment it is possible that the second zone only is such that its external surface presents higher protein adsorption than its inner face.

In another embodiment, the two zones are such that their external surfaces present higher protein adsorption than their inner faces.

Such a device is disclosed in WO 2013/079362. The contents of this patent application are completely applicable to the device that can be used in the method as herein disclosed. In particular, all embodiments disclosed in WO 2013/079362 are applicable to said device.

In particular, said device can present one or more of the following, using all possible combinations:
- the lower ring and the upper ring have openings that allow the device to be sutured to the surrounding tissues by surgery
- it also presents another element situated under the lower ring and made of a rigid or semi-rigid biocompatible material, said element contributing to holding the device according to the invention in position
- the shape of the upper ring is selected in order to receive the shape of the implant or of the prosthesis for which it will provide the support, and said upper ring is equipped with a means for anchoring said implant or said prosthesis
- said anchoring means is selected from among a fixation system providing locking by rotation, by shrink-fit or force-fit assembly, by threading, by fixing with springs (clips), by lugs, by hoops, by countersinking or by interlocking
- the shape of the lower ring, and of the other element (if present), corresponds to the anatomical shape of the tissue for implantation of the device (in particular the trachea)
- it is to be installed *in situ* in or on the trachea of the patient by means of the lower ring
- the upper ring is of a grooved shape.

As mentioned in WO 2013/079362, said medical device is generally intended to receive a removable implant or prosthesis for which it will provide a support, wherein said removable implant or prosthesis will provide the functional repair and replacement of the missing larynx.

The invention also relates to a method for obtaining a prosthesis as described above, comprising the step of modifying the structure of surface of external face of the tubular part of said prosthesis, while not modifying the surface of the internal face.

The modification of the structure of the surface of the external face of the tubular face is intended to obtain one or more of the following:
- increase of protein adsorption on the surface (as compared to a non-modified surface). This can be checked, in particular by BCA assay.
- increase of hydrophilicity of the surface (as compared to a non-modified surface). This can be checked, in particular, by sessile drop contact angle assay with the help of a goniometer.
- decrease in machine marks on the external surface (as compared to the non-modified surface). This can be checked, in particular by electron microscopy or high magnification light microscopy.

In a particular embodiment, said step of modifying the surface of the external face (modifying step) comprises the step of applying an acid etching treatment to said external face.

In another embodiment, said step of modifying the surface of the external face comprises the step of anodizing said external face.

It is most preferred when said treatment is performed so as to limit the treatment effects are limited to certain zones of the implant *via* use of masks and holders made of material resistant to the treatment solution, which would thus prevent the treatment to reach non-desired zones.

In particular, it is most preferred when such masks or holders protect the inner face of the treated tubular part, when letting the outer part be accessible to the treatment.

Consequently, said modifying step is preferably performed on the external face of the tubular part only by use of masks made of material resistant to the treatment solution for the other parts of the prosthesis, including the inner face of the treated tubular part. Such material (such as Teflon™, polypropylene), resistant to the treatment solution components at the given durations and physical conditions (such as temperature) and preventing the movement of the treatment solution to the interior of the implant, will be disclosed below.

It is further indicated that the process may include a step of cleaning the prosthesis before application of the surface treatment. Such cleaning may be performed using any solvent known in the art such as acetone, ethanol (100%, 90% or 70%), or the like. Cleaning may include use of a combination of more solvents.

It is to be noted that the prosthesis may be whole made of titanium, and that the method encompasses the fact that the acid etching treatment is performed on the prosthesis as a whole or on only part of it.

Alternatively, only a part of the prosthesis may made of titanium, and the acid etching treatment may be performed on the whole titanium part of the prosthesis or of only a part of said titanium part.

It is preferred when the etching treatment is performed with a strong acid.

A strong acid is an acid which completely ionizes in a solution of water (i.e. which show complete dissociation when put in water).

Consequently, the strong acid RH (where R represents the associated base) will completely dissociate to R⁻ and H⁺ (actually H₃O⁺) when put in water, whereas a weak acid does completely dissociate (RH coexists with R⁻ and H₃O⁺). The pH of strong acids is generally comprised between 1 and 3 and many strong acids have a negative pKa value.

As strong acids, one can cite: hydrochloric acid (HCl), hydrobromic acid (HBr), hydroiodic acid (HI), sulfuric acid (H₂SO₄) perchloric acid (HClO₄), p-Toluenesulfonic acid (CH₃C₆H₄SO₃H), nitric acid (HNO₃), or chloric acid (HClO₃).

The method may also be performed with hydrofluoric acid which is a solution of hydrogen fluoride (HF) in water. Although this acid is classified as a weak acid because of its lower dissociation constant compared to the strong acids, this acid is highly corrosive and may thus be used in the etching process. In particular, it can be used in water or dissolved in another acid such as acetic acid.

The etching conditions (temperature, duration, acid concentration) can easily be determined by the person skilled in the art.

The temperature of the reaction is comprised between 15°C and 80°C. Although high temperatures (higher than 50°C) can be used, it is generally preferred when the etching treatment is performed at room temperature (i.e. between 15°C and 25°C).

The duration of the reaction will primarily depend from the nature of the acid used and its concentration. It would generally be comprised between 1 minute and 24 hours.

For highly corrosive acids such as HF, treatments in the range of minutes would be performed for ensuring the structural stability. For other acids such as HCl, periods in the range of hours can be used. However, in order to achieve good result and have a good industrial process, the duration of the etching treatment will preferably be lower than 12 hours, more preferably lower than 6 hours, more preferably lower than 3 hours, or lower than 2 hours. It is generally higher than 10 minutes, more preferably higher than 15 minutes, or higher than 20 minutes.

The duration of the etching treatment may thus generally be higher than 30 minutes. It would also be generally lower than 1h30. However, and as reminded above, this duration is very dependent on the acid used and its concentration. The person skilled in the art may adapt the appropriate duration by measuring the contact angle of the titanium, the adsorption of proteins, and also the visual changes in etching solution (Color change, excessive material presence in the solution particularly from the porous part etc.) as described below, in order to determine whether the surface of the titanium has been properly modified.

Another visual control method is the determination of the color change of the implant during the treatment and microscopic observation of the disappearance of the machining marks as a function of treatment. These controls can be done with respect to pre-established reference images and/or samples for a given set of parameters.

The process of the invention shall preferably be carried out according to the following parameters:

| Acid used | Concentration (M) | Time of exposure of titanium to the acid |
|---|---|---|
| HCl | 5-15M, preferably 10 M | 30 min - 24 h, preferably around 1 h |
| H₂SO₄ | 2-5M, preferably 3M | 5 min - 2 h, preferably around 30 minutes |
| HF | 5-15M, preferably 9 M | 1 min - 5 min, preferably around 1 minute |

In the above table, the term "around" shall mean +/- 10%.

The prosthesis are generally prepared before being exposed to the acid solution. They are, in particular, washed and decontaminated.

The washing and decontamination steps include bathing the prosthesis in acetone and in ethanol (70-100%) and washing the prosthesis between and after the baths with water (in particular ultrapure water, such as MilliQ® water (Millipore). Ultrapure water usable in the context of the process is characterized by a resistivity around 18.2 MΩ·cm at 25 °C and a conductivity at 25 °C of around 0.056 µS·cm⁻¹.

One can cite a process with a bath of acetone (30min-1h30) followed by washing (dipping in water), and ethanol (70%, 30min-1h30)), followed by a dip in water and bath in water (30min-1h30). The prosthesis may dry at room temperature and can then be used for the etching treatment (Figure 2).

Said treatment shall include a bath in the acid solution under constant agitation which will generally be followed by various washed with water (especially ultrapure water), by quickly dipping the prosthesis in water and/or bathing them.

The treated prosthesis can later dry at room temperature and be sterilely conditioned.

In another embodiment, said step of modifying the surface of the prosthesis is anodization (anodizing step).

Anodization is an electrolytic passivation process used to increase the thickness of the natural oxide layer on the surface of metal parts.

It is preferred when said anodizing step is performed in Hydrofluoric Acid 0,5% ,/Acetic Acid 98% at room temperature. In a preferred embodiment, said anodizing step is performed at a voltage from 10 to 25 V, preferably around 20V.

In a preferred embodiment, said anodizing is performed for 10 to 60 minutes preferably around 20 minutes.

In the above, "around" means +/- 10%.

However, other anodization protocols may be developed, as long as they lead to the same prosthesis properties.

As demonstrated in the examples, the etching or anodization treatments will modify the surface of the titanium and increase the hydrophilicity. This can be shown by a decrease of the water contact angle, which is the angle between the surface, here treated or non-treated titanium and the line tangent to the curve formed by the water drop on the said surface. This is measured by a 10 microliter water droplet by a contact angle goniometer, as described above.

As demonstrated in the examples, the etching or anodization treatments will modify the surface of the titanium and increase the adsorption of proteins, in particular of serum proteins. This can be shown by the measurement of the amount of protein adsorped by Bradford Assay (BCA, as described above). It is to be noted that, for more precise determination, the proteins in the desorption solution can be analyzed by running the samples in HPLC. The examples clearly showed a significant increase in the protein adsorption for the outer surface of the prosthesis.

The treated samples had significantly higher amount of protein adsorbed relative to the untreated samples. Consequently, the treated surfaces will better interact with the serum, which is the first step in the interaction between the implant and tissue.

To observe the effect of acid treatment on the interaction of the implant / tissue *in vivo,* the treated and untreated samples were implanted subcutaneously in rats. After explantation it was observed that the penetration and coverage of the samples processed by the surrounding tissue was significantly higher.

Consequently, the acid etching treatment with a strong acid solution, or HF or the anodization treatment will modify the surface in three significant areas
i) modification of nanoscale roughness
ii) changes increasing hydrophilicity
iii) improved protein adsorption

Improved protein adsorption is considered to be the most critical improvement, as it directly relates to a better interaction with the serum and thus to improved *in vivo* integration.

Consequently, the treated prosthesis presents a different structure than the non-treated prosthesis, in particular the ones disclosed in WO 2013/079362, where no surface treatment is envisaged, and where good *in vivo* integration is only planed through a specific surgical procedure.

After such disclosed acid etching or anodization treatment, said prosthesis can be implanted in a patient. This comprises the step of surgically fixing said prosthesis to the walls of the trachea of a patient who has undergone a laryngectomy.

Said acid etching or anodization treatment shall improve colonization of the implanted prosthesis by the connective tissue of the implantation site, and shall thus improve recovery of the patient and decrease the adverse effects.

Using the process and the prosthesis as disclosed, it is thus possible to treat a patient who has undergone a laryngectomy.

A method for treating a patient having undergone laryngectomy could comprise
- the step of surgically fixing an acid-treated prosthesis to the walls of the trachea, wherein said prosthesis comprises two zones, one of which constituting an upper ring made of a rigid or semi-rigid, solid titanium, and the other of which constituting a lower ring made of a rigid or semi-rigid, porous titanium, and
- the further step of inserting an implant through the mouth of said patient and fixing said implant onto said prosthesis.

In a specific embodiment, said implant comprises two flaps (82, 83) and also two hinges (84, 85), or wherein said implant comprises a tubular body having a proximal end (6) surrounding a proximal opening, a flap having a proximal end (6) and a distal end, a hinge linking the proximal end of the flap to the proximal end of the tubular body, so that the flap can be placed in a normal position where it covers the proximal opening, and in an open position where it does not cover the proximal opening, and wherein the flap is such that the distal end of the flap forms a flange covering a part of the tubular body, and a spacing exists between said distal end of the flap covering said part of said tubular body and said tubular body to allow air to enter the proximal opening through said spacing.

In another embodiment, said implant may comprise one or more of the following characteristics, as described in PCT/EP2014/068913
- it comprises a tubular body having a proximal end surrounding a proximal opening, a flap (or valve) having a proximal end and a distal end, a hinge linking the proximal end of the flap to the proximal end of the tubular body, so that the flap can be placed in a normal position where it covers the proximal opening, and in a open position where it does not covers the proximal opening, and wherein the flap is such that the distal end of the flap forms a flange covering a part of the tubular body, and a spacing exists between said distal end of the flap covering said part of said tubular body and said tubular body to allow air to enter the proximal opening through said spacing
- the flap has a shape, so as to avoid the stagnation of the bolus of the saliva and on the flap, and have these elements discharged by gravity
- the tubular body presents two zones:
   o a first zone which may be inserted in whole or in part in the larynx of a patient or fixed on the supporting device as described above and in WO 2013/079362
   o a second zone (including the proximal end) which presents external dimensions smaller than the external dimensions of the first zone (so that one passes from the first zone to the second zone by a narrowing of the external dimensions of the tubular body). This second zone can be truncated or wedge-shaped (or shaped as a whistle tip or as the nozzle of a recorder).

It is further reminded that such supporting device and such implants are described, in particular in EP 2240120 and WO2013079362, as well as a specific surgical method to implant such. Furthermore, WO 2014/079904 discloses devices for fitting and removing such prosthesis through the mouth.

### DESCRIPTION OF THE FIGURES

Figure 1: Photograph of a droplet in a contact angle goniometer.
Figure 2: Streamline of the process. Upper line: cleaning of the prosthesis. Lower line: acid etching process
Figure 3: Acid-etched surface observed by SEM (Scanning Electron Microscopy)
Figure 4: Description of the method of analysis/quantification of the effect of acid etching *via* image analysis
Figure 5: Protein Adsorption to the surfaces as observed by BCA assay
Figure 6: Observation of explanted porous titanium implants after subcutaneous implantation by SEM (Scanning Electron Microscopy)
Figure 7: Collagen and total protein amount: NTS: non-treated; HCL: acid etching with HCl; ANO: anodization treatment
Figure 8: Treatments and cell culture configurations utilized in example 1.2.6.

### EXAMPLES

These examples report, in particular, effects of the surface modifications on *in vivo* soft tissue infiltration into the porous implants observed by a rat subcutaneous implantation model for 2 weeks.

In order to elucidate the reasons for the beneficial effects of nanoscale surface treatments on *in vivo* integration, titanium disks made of titanium microbeads of 300-500 microns in diameter were used, to study the effect of surface modifications on human fibroblast cell behavior with respect to several parameters which are known to effect cell attachment, namely surface hydrophilicity, roughness and serum protein adsorption.

The nature and the content of proteins adsorbed was quantified by HPLC separation and subsequent protein sequencing to better understand the interaction between surface property changes and protein retention. Different cell culture configurations were used to mimic different in vivo conditions, such as mimicking cell in-growth from surrounding soft tissues by encapsulation of single titanium beads in cell-laden gelatin hydrogels.

### Example 1. Materials and Methods:

### 1.1 Materials.

Pure Medical Grade Titanium beads (Ti40) were purchased from Neyco SA (Paris, France). All Chemicals were purchased from Sigma-Aldrich (Germany) unless otherwise stated. Primary Vimentin antibody was purchased from Santa-Cruz (California, USA). Vybrant® cell adhesion assay was purchased from Life Technologies (USA). RNeasy® mRNA extraction kit was purchased from Quiagen (The Netherlands) and iScript® Reverse transcription Supermix for RT-qPCR was obtained from Bio-Rad. Apoptotic/Necrotic/Live cell assay was purchased from PromoKine (Heidelberg, Germany).

### 1.2 Methods

### 1.2.1 Surface Treatments

Surface modification of the plain titanium and 3D porous titanium samples were done as described below (Table 1). After the treatments samples were washed with distilled water and air-dried.

| **Type of treatment / Abbreviation** | **Concentration** | **Duration** | **Temperature** |
|---|---|---|---|
| Hydrochloric Acid / HCl | 10M | 1 hour | Room Temperature |
| Anodization / ANO | Hydrofluoric Acid 0,5% , Acetic Acid 98% | 30 minutes | Room Temperature |
| Fetal Bovine Serum / FBS | - | 1 hour | Room Temperature |

### 1.2.2 In vivo tests

Nine male Wistar rats weighing 300g were implanted. Approval for those experiments was obtained from the Regional Committee of Ethic for Animal Experiments of Strasbourg. The implantation procedure was done under general anesthesia, obtained with intra peritoneal injection of a mixture made with 90 mg/kg of ketamine and 10 mg/kg of xylasine. Local anesthesia of the skin of the back was performed. Implants were put subcutaneously in the back of the animals. The skin was stitched up with several stitches. Each animal received up to six implants (in most cases, two at each side of the spinal column). Animals were daily observed to see their well-being and whether there were infection signs on the operation area. Two weeks after implantation procedure, animals were anaesthetized and the implants were explanted.

Macrophotographies of the implants were performed. Afterwards, implants were coated with a 10 nm gold layer to be observed with SEM, the total coverage after 2 weeks for surface and crossection for each condition was quantified using Image J program available at http://imagej.nih.gov/ij/.

Total amount of protein and collagen was quantified by semi-quantitative Fast Green-Sirius Red staining. Explants with the in-grown tissue were immersed in 30 mg/ml protease solution in sterile MilliQ® water (Origin of protease was *Streptomyces griseus).* For total digestion of the ingrown tissues, up to 48 hours of incubation was necessary.

mRNA was isolated by RNeasy® kit as per instructions of the provider. The amount of extracted RNA and its quality was measured by a Nanodrop® spectrometer system (Thermo Scientific). The reverse transcription of the complementary DNA was done by Reverse transcription Supermix® for RT-qPCR (Bio-Rad), by the following cycle configuration: 5 min at 25°C, 30 min at 42°C and 5 min at 85°C. The amplication was done with the following reaction solution composition: 4 µL of supermix at 5 M, 0,1 µL of forward primer 0,1 µL of reverse primer, 0,2 µL of Taq polymerase (iTaq DNA polymerase, Bio-Rad), 4 µL of Bétaïne at 5 M, 1 µL of DMSO, 9,6 µL filtred MilliQ® water and 1 µL complementary DNA. The amplification cycle was as follows: 1 min at 94°C, 1 min at 60°C, then 1 min at 72°C for 35 times. The primers were designed by the primer design tool of the primer provider (Eurofins MWG, Operon).

The expression level of the following genes were checked: Interleukin-10 (IL-10), Tissue inhibitor of Matrix metalloprotease 1(TIMP-1), von Willebrand factor (vwf), Vascular endothelial growth factor A (VEGF A), Tumor necrosis factor alpha (TNF-α), Cadherin 1 (Cdh-1), Vascular Cell Adhesion Molecule 1 (VCAM-1), Interferon gamma (IF-γ), Transforming Growth Factor beta (TGF-ß), Glyceraldehyde-3-phosphate dehydrogenase (Gapdh), beta actin (ß-actin) with the aim of comparing if there is a significant difference in the pro and/or anti-inflammatory and vascularization related markers.

### 1.2.3 Surface Characterization

The surfaces of the titanium implants were characterized for the morphology (SEM), roughness (AFM), topography (AFM) and surface hydrophilicity (Contact Angle Measurement). The effect of the treatments on surface roughness was quantified by AFM in contact mode in 10 micron x 10 micron fields (Multimode Nanoscope VI, Bruker (Santa Barbara, CA, USA)). Surface roughness was calculated from an average of 6 images. Contact angles of the treated and non-treated surfaces were done by the measurement of contact angle for a 10 microliter water droplet as described above Protein adsorption was initially quantified by BCA assay and further characterization was done as described below.

### 1.2.4 Quantification of Serum Protein Adsorption via HPLC

In order to quantify the amount of protein adsorbed, single beads of each condition was incubated in human serum at room temperature for 1 hour. After the incubation the serum was removed and introduced to the HPLC system to quantify the missing amount of protein components which have been adsorbed. For the baseline human serum was directly introduced into the HPLC system.

### 1.2.4.1 - RP-HPLC purification

The proteins present in samples (100 µL) were separated at 40°C using a Dionex HPLC system (Ultimate 3000; Sunnyvale, CA USA) on a Vydac 208TP C8-column (particle size, 5 µm ; 150 mm x 2.1 mm ; Grace, Vydac, Interchim, Montlugon, France) with a pre-column heater 2 µL and 0.13 mm for internal diameter (Interchim, Montluçon, France). Absorbance was monitored at 214 nm, and the solvent system consisted of 0.1 % (v/v) TFA in water (solvent A) and 0.09% (v/v) TFA in 70% (v/v) acetonitrile-water (solvent B). Elution was performed at a flow rate of 200 µL/min with the following solvent B gradient: 0%B during 5 min, 0%B to 100%B during 45 min and 100%B during 10 min. Fractions containing peaks were manually collected and concentrated until 20 µL by evaporation using a speed-vac.

### 1.2.5 Cell Culture

Primary Human Gingival Fibroblasts isolated in University of Strasbourg Dental Faculty with patient consent were used at passage 3-6. The medium was DMEM low glucose with 10% FBS and 1% Pen/Strep. Cells were passaged regularly and fed every other day. Trypsinized cells were seeded on single beads which are sterilized by UV treatment. For bead suspension experiments non-adhesive cell culture plates were used (NUNC). 1 x 105 cells were seeded on each bead. For encapsulation 5% porcine gelatin solution was used. 1.2 x 105 cells were encapsulated within the gel which contains a single titanium bead inside by introduction of transglutaminase to crosslink gelatin (6:1 v:v ratio).

### 1.2.6 Cell Culture Configurations

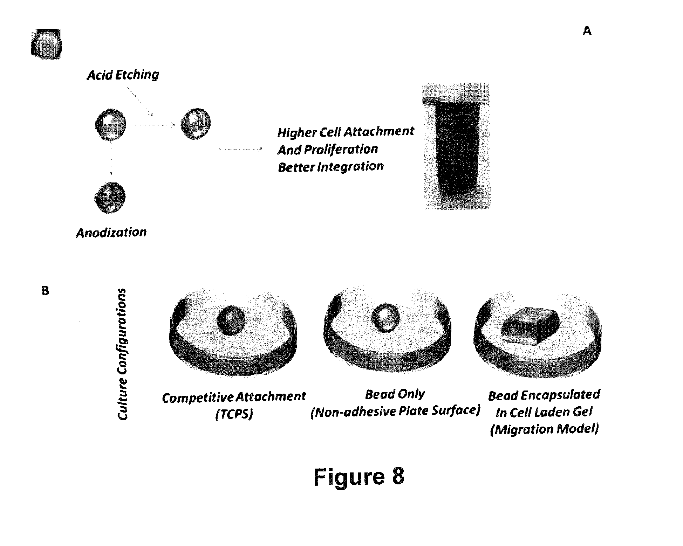

Cell culture experiments were done in 3 configurations as described in Figure 8.

First, to see the competitive attachment of the cells, cells were seeded on beads on TCPS where they can attach both the well and the titanium bead.

In the second configuration, to see the preference of the cells to attach on the beads, a non-adhesive plate was used, so that the cells can only attach to the beads.

The third configuration was the encapsulation of the beads in a cell laden enzymatically degradable hydrogel (gelatin) to mimic the movement of the cells from the surrounding tissue to the implant.

### 1.2.7 Cell Viability, Morphology and Distribution

Initial Cell attachment and viability over 5 days was tested with TOX-8 (A resazurin based cell metabolic activity test). Cellular morphology and cell distribution was either observed by Calcein-AM staining or Hoechst/Phalloidin/Vimentin staining. 3D imaging of single beads in every configuration was done by a confocal microscope after Calcein-AM staining. Total cell area and total cell numbers for each conditions were determined by Image-J program.

### 1.3 Statistical Analysis

The comparisons between, non-treated, acid etched and anodized conditions was done by one-way ANOVA analysis at a statistical significance level of p<0.05, followed by Tukey's test to determine the differences in between samples.

### Example 2. Results

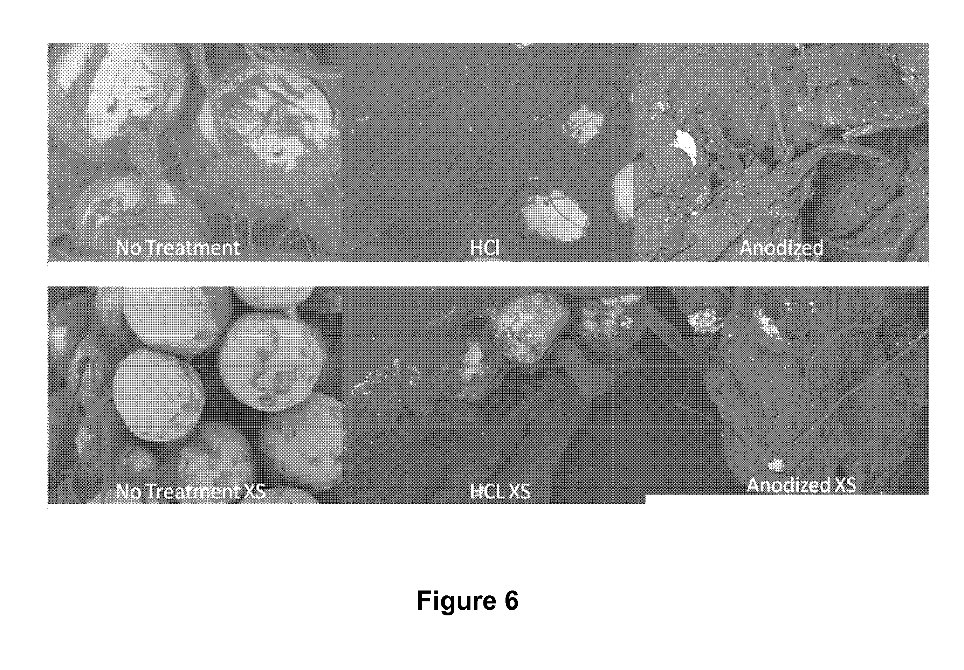

The surface treatments had profound effects on the integration of the implants *in vivo* (Figure 6).

After two weeks of subcutaneous implantation in rats, the extent of tissue formation around the implants was considerably higher in surface treated samples. SEM images demonstrated that both on the surface and in the cross-section; there was considerably more cell infiltration and extracellular matrix for both anodized and HCl treated samples. Particularly, the amount of tissue in the cross-section was significantly higher.

An increased expression of Interleukin-10 (IL-10) and Tissue Inhibitor of Matrix Metalloprotease 1 (TIMP-1) were also observed by Rt-PCR in anodized samples, whereas there was no significant difference in the expression of the other genes checked between the groups.

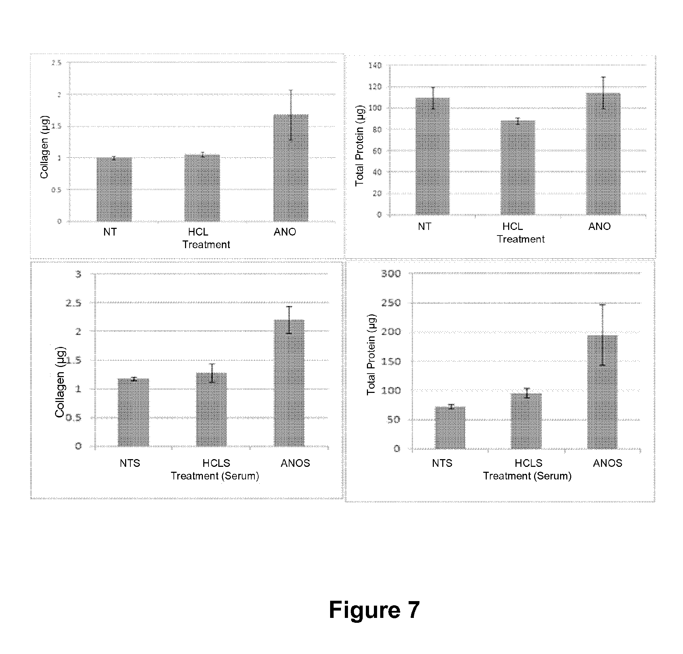

By introducing rat serum onto the implants prior to the implantation, the protein adsorption and subsequently facilitation of the tissue integration was induced (as a proof of concept). This treatment resulted in additional amelioration for the treated surfaces, with significantly more colonization. When the total area covered by tissue for both the surface and the cross-section of the implants were quantified, we observed that the treated implants had significantly more coverage in all configurations. Also, in treated samples there was a significantly higher amount of collagen secretion (Figure 7).

Anodization locally induced microlevel changes but its effect was mainly at nanometer level with production of a regular array of titanium oxide nanotubes . The size of nanotubes was 50±16 nm having a size suitable for deposition of proteins.

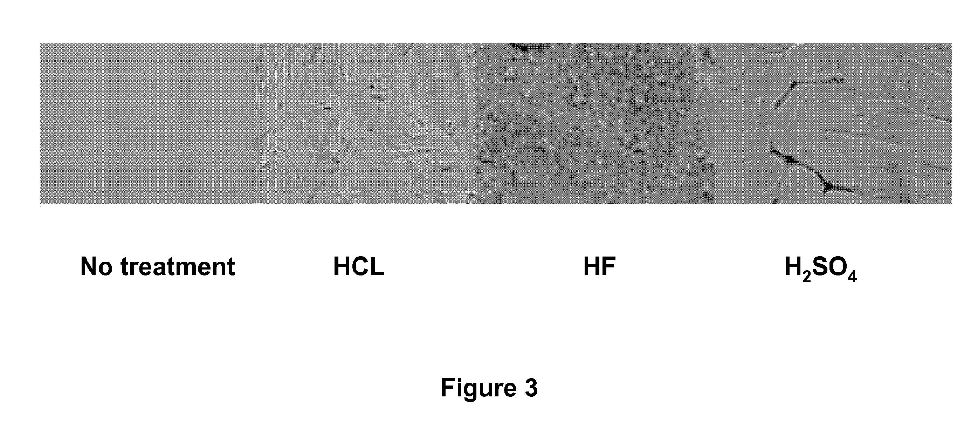

We have selected HCl etching due to the milder conditions of etching. Utilization of different acids lead to different surface morphologies for the beads, for example HF resulted in significant change in bead morphology whereas HCl and H₂SO₄ treatments where mainly limited to the surface changes (Figure 3)..

Surface treatments improved the hydrophilicity of pure titanium which has a contact angle of 66.2 ± 3.6 °. Both anodization and acid etching decreased the contact angle, i.e. increased the hydrophilicity. This had a direct effect on protein adsorption on the microbeads also. We have quantified the adsorption of proteins from human serum. When human serum is directly used, acid etched samples adsorped more proteins as quantified by HPLC. (Figure 5). The main proteins that have been adsorbed were albumin, transferrin, Apolipoprotein A and Alpha1 antitrypsine and vitamin D binding protein. After depletion of IgG and Albumin, again there was a significant difference in the absorption of Apolipoprotein and Alpha 1 antitrypsine. For anodized samples the absorption trend was similar although the adsorption was less compared to HCl treated surfaces.

After 5 days of culture, the surface of the treated beads were mostly covered by cells in suspension culture whereas the non-treated beads had less cells due to the lack of initial attachment. For gel culture, cells preferentially moved towards the beads; most probably due to its stiffness. This cell migration was more pronounced in the cases of anodized and HCl treated beads. In the case of TCPS surfaces, the cells became confluent and the beads were covered for all cases. When analyzed with confocal microscopy the superior cell coating on treated samples were more apparent for all conditions except TCPS

### Example 3. Discussion

One of the primary effects of both etching and anodization is the alteration of titanium surface topography at both nano- and micro levels. Topographical changes even at the level of 10 nm can affect cell behavior.

It has been recently shown that the presence of surface micro- and/or nano-topographies can down regulate inflammatory response (Palmquist *et a*l.) with a significant decrease in both polymorphonuclear and mononuclear cells in rat subcutaneous implantation experiments. The decrease in the initial response due to the surface structuration might contribute to the faster integration of 3D porous structures too.

For stem cells, it has been shown that the presence of nanoscale cues might be enough by itself for successful commitment of cells to a specific differentiated cell type (McNamara *et al*.). Nanoscale cues can also provide surfaces with different preference to bacterial and mammalian cell attachment (Wang *et al*.). For osteoblasts, the positive effect of both acid etching and anodization is well recorded and a recent study showed that while both anodized and acid etched surfaces resulted in up-regulation of osteoblast differentiation related genes, anodized surfaces also caused an increase in cell adhesion related genes (Sista *et al.).* Thus the increase in the nanoscale cues on the surfaces of the titanium beads can provide a better surface for cell attachment.

At the coating level, it has been shown that rough titanium surfaces can decrease fibroblast activity while increasing osteoblast activity (Cohen *et al.).*

But for bulk materials, especially surface modified materials, the effect is not only related to the roughness, thus the fibroblast behavior is more varied.

For example, as the roughness results for the acid etched and anodized beads shows before and after serum incubation, the changes in surface topography affects the protein absorption and protein adsorption in return affects the surface roughness. For HCl etched beads due to the increased protein absorption there was an increase in the surface roughness, whereas most probably due to the filling of the nanopores the effect was reversed for anodized samples. These might result in differential integrin clustering due to the distribution of the proteins on the implant surface. But even though the anodized samples have lower protein adsorption and roughness compared to acid etched samples, the size of the nanotubes exert additional and more precise effects on cell/surface interaction. The protein absorption process goes through the filling of the nanopores and it has been shown that with pores greater than 100 nm this may not be achieved; but for pores of 50 nm they can be completely filled (Brammer *et al*.).

The method and nature of nano/micro structuration is also an important determinant. For example laser assisted structuration methods can result in highly hydrophobic surfaces, even though the surface is highly hydrophilic immediately after the treatment (Kenar *et al*.), due to the regular nature of the surface topography, without a chemical change at the interface level (where the chemical effect of the treatment is only applicable in the direct treatment areas with the laser).

For the bulk materials, protein adsorption seems to be a more determinant factor than surface roughness. For example, if the roughness is obtained by grounding the reverse effect and improved fibroblast response has been reported (Eisenbarth *et al*.). Moreover, in a planar surface it is easier to single out the effect of roughness as the cells only interact with the surface from the top. For human gingival fibroblasts formation of a hydride layer was shown to be an effective way of improving attachment (Xing *et al*.), however again it was not possible to distinguish the source of this effect with respect to surface roughness or contact angle. It was suggested that the effect of the hybrid layer is to improve the protein absorption.

In the above example, it was also shown that the surface treatments are able to increase the protein absorption on the porous titanium surfaces. The quantification of the specific proteins that are preferentially adsorbed to a given surface can control the initial phases of tissue response to the implant (Nakanishi *et al*.).

Formation of an initial serum albumin layer on implants is a common occurrence due to the relative abundance of serum albumin. However, the extent of this initial layer can facilitate the absorption of other proteins *in vivo,* thus a higher serum albumin absorption can be advantageous, as in the case of treated surfaces in this study. As an illustration, one of the proteins identified in this study, Apolipoprotein, has been implicated in having an effect in regeneration and also in cell growth and proliferation (Mahley), but it has been also related to inhibition of endothelial cell growth. Whereas, Alpha 1 Antitrypsin is a protease inhibitor that can increase the retention time of the other adsorbed proteins due to its anti-protease activity (Gettins *et al*.).

Most of the studies on the effect of surface properties on cells are done on planar surfaces which are hard to convert into information in 3D configurations. In a 3D metallic foams cells are exposed to significantly curved surfaces which has been shown to affect their ability to move and spread by affecting the contractile forces exerted by the cell cytoskeleton (Sanz-Herrera *et al.).*

Moreover, analysis of the 3D titanium structures especially with conventional methods is difficult. Titanium microbeads are small enough to be visualized by fluorescence microscopy or confocal microscopy, which provides an opportunity to observe the cellular behavior in a more 3D microenvironment. Moreover, bead based structures are advantageous as they provide macroscopically smooth surfaces that interact with the surrounding tissue unlike many metallic foams which has sharp edges that can damage the tissues in vicinity. Also physical properties such as porosity or pore size can be easily modified by using the mixture of beads with different granulometry. Previously polymeric beads has been used in delivery and immunoisolation of the cells as they provide an excellent surface:volume ratio and a beneficial effect on mass transfer limitations (Khademhosseini *et al.*).

The effects of surface treatments *in vivo* were much more pronounced compared to *in vitro* results.

This is due to the incremental effects of increased hydrophilicity and roughness and resulting protein absorption. As the biomaterial interaction with the body is mainly governed by the initial interaction with the body fluids, mainly blood, and subsequent protein adsorption it is important for *in vivo* application to utilize this interactions beforehand. The blood in the surgery area is in direct contact with wounded endothelial cells and will most probably contain high amounts of pro-inflammatory signals; thus its adsorption on the surface of the implant not only will trigger coagulation but also will result in a more pro-inflammatory initial response.

However, if the implant has already been partially coated with autologous serum from peripheral blood; then this effect can be diminished. As the surface treatments increase the protein adsorption this would be a good way to boost the colonization also. The *in vivo* tests showed that for all conditions application of serum caused a significant increase in colonization.

The effects of two surface modification techniques, acid etching and nanotube formation via anodization, was quantified in a 3D model. The sum of all effects of surface treatments that are beneficial for cell attachment such as increased surface roughness, improved protein absorption has been shown to have an exponential impact to the in vivo integration. These treatments will be tested for improvement of colonization of porous titanium tracheal connectors in artificial larynx implants.

### REFERENCES

1. Li et al. Bone ingrowth in porous titanium implants produced by 3D fiber deposition. Biomaterials 2007;28:2810-20.
2. Vrana et al. Modification of macroporous titanium tracheal implants with biodegradable structures: Tracking in vivo integration for determination of optimal in situ epithelialization conditions. Biotechnology and Bioengineering 2012:n/a-n/a.
3. Janssen et al. Laryngotracheal reconstruction with porous titanium in rabbits: are vascular carriers and mucosal grafts really necessary? Journal of Tissue Engineering and Regenerative Medicine 2010;4:395-403.
4. Dupret-Bories A, Schultz P, Vrana NE, et al.. Development of surgical protocol for implantation of tracheal prostheses in sheep. Journal of Rehabilitation Research and Development 2011;48:851-63.
5. Gaafar et al. Laryngotracheal augmentation using titanium mesh. The Journal of Laryngology & Otology 2008;122:391-96.
6. Lauer et al. The titanium surface texture effects adherence and growth of human gingival keratinocytes and human maxillar osteoblast-like cells in vitro. Biomaterials 2001;22:2799-809.
7. Variola al. Improving Biocompatibility of Implantable Metals by Nanoscale Modification of Surfaces: An Overview of Strategies, Fabrication Methods, and Challenges. Small 2009;5:996-1006.
8. Cho S-A, Park K-T. The removal torque of titanium screw inserted in rabbit tibia treated by dual acid etching. Biomaterials 2003;24:3611-17.
9. Zhao et al. High surface energy enhances cell response to titanium substrate microstructure. Journal of Biomedical Materials Research Part A 2005;74A:49-58.
10. Mustafa et al. Determining optimal surface roughness of TiO2 blasted titanium implant material for attachment, proliferation and differentiation of cells derived from human mandibular alveolar bone. Clinical Oral Implants Research 2001;12:515-25.
11. Vrana et al. Multi-Scale Modification of Metallic Implants With Pore Gradients, Polyelectrolytes and Their Indirect Monitoring In vivo. 2013a:e50533.
12. Vrana et al. Titanium Microbead-Based Porous Implants: Bead Size Controls Cell Response and Host Integration. Advanced Healthcare Materials 2013b:n/a-n/a.
13. Takemoto et al. Osteoinductive porous titanium implants: Effect of sodium removal by dilute HCl treatment. Biomaterials 2006;27:2682-91.
14. Lavenus al. Adhesion and osteogenic differentiation of human mesenchymal stem cells on titanium nanopores. Eur Cell Mater 2011;22:84-96.
15. Lavenus et al. Cell differentiation and osseointegration influenced by nanoscale anodized titanium surfaces. Nanomedicine 2012;7:1045-66.
16. Palmquist et al. Acute Inflammatory Response to Laser-Induced Micro- and Nano-Sized Titanium Surface Features. Clinical Implant Dentistry and Related Research 2013;15:96-104.
17. McNamara et al. Nanotopographical Control of Stem Cell Differentiation. Journal of Tissue Engineering 2010;1.
18. Wang et al. Length□Scale Mediated Differential Adhesion of Mammalian Cells and Microbes. Advanced Functional Materials 2011;21:3916-23.
19. Sista Set al. Cell biological responses of osteoblasts on anodized nanotubular surface of a titanium□zirconium alloy. Journal of Biomedical Materials Research Part A 2013.
20. Cohen et al. Decreased fibroblast and increased osteoblast functions on ionic plasma deposited nanostructured Ti coatings. Nanoscale Research Letters 2007;2:385-90.
21. Brammer et al. Biomaterials and Biotechnology Schemes Utilizing TiO2 Nanotube Arrays. Biomaterials Science and Engineering ISBN:978-53.
22. Kenar et al. Femtosecond laser treatment of 316L improves its surface nanoroughness and carbon content and promotes osseointegration: An in vitro evaluation. Colloids and Surfaces B: Biointerfaces 2013;108:305-12.
23. Eisenbarth et al. Influence of the surface structure of titanium materials on the adhesion of fibroblasts. Biomaterials 1996;17:1399-403.
24. Xing et al. Surface hydride on titanium by cathodic polarization promotes human gingival fibroblast growth. Journal of Biomedical Materials Research Part A 2013:n/a-n/a.
25. Nakanishi et al. On the adsorption of proteins on solid surfaces, a common but very complicated phenomenon. Journal of bioscience and bioengineering 2001;91:233-44.
26. Mahley RW. Apolipoprotein E: cholesterol transport protein with expanding role in cell biology. Science 1988;240:622-30.
27. Gettins PGW. Serpin Structure, Mechanism, and Function. Chemical Reviews 2002;102:4751-804.
28. Sanz-Herrera et al. On the effect of substrate curvature on cell mechanics. Biomaterials 2009;30:6674-86.
29. Khademhosseini et al. Conformal coating of mammalian cells immobilized onto magnetically driven beads. Tissue Engineering 2005;11:1797-806.
30. Kunzler et al., Systematic study of osteoblast and fibroblast response to roughness by means of surface-morphology gradients, Biomaterials, Volume 28, Issue 13, May 2007, Pages 2175-2182)

## Claims

1. A prosthesis comprising a tubular part made of titanium, wherein said tubular prosthesis presents protein absorption on the external face of said tubular part that is higher that the protein absorption on the internal face of said tubular part.

2. The prosthesis of claim 1, wherein said protein absorption on the external face is higher of at least 20 % than the protein absorption on its internal face.

3. The prosthesis of claim 1 or 2, wherein said titanium is porous titanium.

4. The prosthesis of any one of claims 1 to 3, wherein said external face of the tubular part exhibits a contact angle lower than 60°.

5. The prosthesis of any one of claims 1 to 4, wherein said prosthesis exhibits a quantifiable decrease in machining marks on the surface of the exterior face of the tubular part and no changes on the surface of the interior part.

6. The tubular prosthesis of any one of claims 1 to 5, which is an laryngeal or tracheal prosthesis.

7. The prosthesis of any one of claims 1 to 6, which consists in a medical device for supporting an implant or prosthesis, wherein said device comprises two zones, one of which constituting an upper ring made of a rigid or semi-rigid, solid titanium, and the other of which constituting a lower ring made of a rigid or semi-rigid, porous titanium.

8. A method for obtaining a prosthesis according to any one of claims 1 to 7, comprising the step of modifying the structure of surface of external face of the tubular part of said prosthesis, while not modifying the surface of the internal face.

9. The method according to claim 8, wherein said step of modifying the surface of the external face comprises the step of applying an acid etching treatment to said external face.

10. The method of claim 9, wherein said acid etching treatment is performed with an acid chosen in the group consisting of HCl, H₂SO₄, HNO₃, HClO₄, HClO₃ and HF.

11. The method of any one of claims 8 to 10, wherein, when said acid is HCl, it is used at a concentration of around 10 M, when said acid is H₂SO₄, it is used at a concentration of around 3M, when said acid is HF it is used at a concentration of around 9 M.

12. The method of claim 8, wherein said step of modifying the surface of the external face comprises the step of anodizing said external face.

13. The method of claim 12, wherein said anodizing step is performed at a voltage comprised between 10 and 25 V.

14. The method of any of claims 8 to 13, wherein said modifying step is performed on the external face of the tubular part only by use of masks made of material resistant to the treatment solution for the other parts of the prosthesis.

15. The method of any of claims 8 to 14, wherein said prosthesis is a tracheal or laryngeal prosthesis.
